# EUROPEAN PATENT SPECIFICATION

(11) **EP 1 520 552 B1**
(45) Date of publication and mention of the grant of the patent: **30.07.2008**
(21) Application number: 04256004.5
(22) Date of filing: 29.09.2004
(51) Int. Cl.: A61F 2/00

(54) **Implantable surgical mesh**
Implantierbares chirurgisches Netz
Implant chirurgical à mailles

(30) Priority: 30.09.2003 US 507191 P
(43) Date of publication of application: 06.04.2005
(73) Proprietor: ETHICON, INC., Somerville, NJ 08876-0151 (US)
(72) Inventor: Kammerer, Gene W., East Brunswick, NJ 08818 (US)
(74) Representative: Mercer, Christopher Paul

(56) References cited:
- EP-A- 0 334 046
- EP-A- 1 306 061
- WO-A-02/087468
- GB-A- 1 008 193
- US-A- 4 633 873

## Description

### BACKGROUND OF THE INVENTION

### 1. Field of the Invention

The present invention relates generally to implantable surgical meshes, and more particularly, to implantable surgical meshes that contain both absorbable and non-absorbable portions in a configuration such that, following absorption of the absorbable portions, the mesh becomes discontinuous in a predetermined direction.

### 2. Background Discussion

Implantable surgical meshes have been widely used for a variety of different surgical procedures such as hernia repair, pelvic floor repair, urethral slings for treating incontinence, and many others. A woven or knitted mesh structure is desirable in that it allows tissue ingrowth into and through the mesh. The tissue ingrowth is in the form of a tissue fibrosis, where non-oriented tissue cells invade the mesh and grow in a random, disorganized fashion. The combination of mesh and ingrown tissue, however, produces a relatively hard, inflexible construction that does not resemble the tissue structure that it is reinforcing or replacing. This is due, in part, to the fact that the mesh structure in combination with the random ingrowth pattern of the tissue does not reflect the natural, organized cell structure in the absence of the foreign body (mesh). Thus, the resulting relatively inflexible structure can lead to tissue erosion problems in proximity to the implant and/or to organs in the vicinity of the implant.

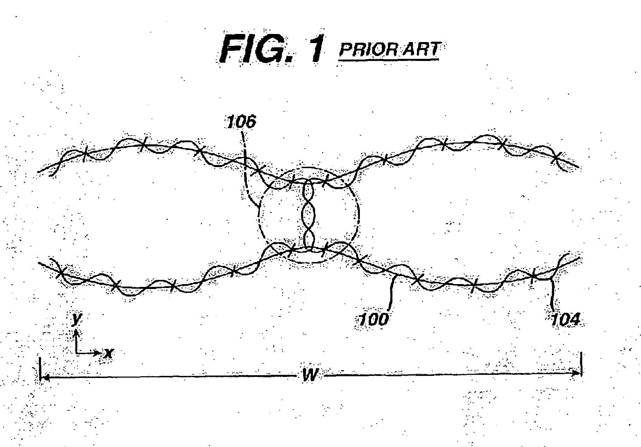

To alleviate these problems, it is known to reduce the amount of tissue ingrowth and decrease the rigidity of the implant by adding absorbable fibers to an otherwise non-absorbable mesh. One such mesh is Vypro®, which is manufactured by Ethicon, Inc. of Somerville, N.J. This mesh is comprised of a combination of about equal parts of polyglactin polymer filaments and polypropylene filaments. When the polyglactin absorbs, it significantly reduces the amount of mesh that remains within the body, leaving only the polypropylene behind. Fig. 1 provides a closer look at the mesh structure of Vypro®. As illustrated, the absorbable polyglactin filaments 100 are positioned next to one another and follow a somewhat sinusoidal path along the entire width of the mesh, or along the x-axis of Fig. 1. The non-absorbable polypropylene filaments 104 are woven more tightly around the individual polyglactin filaments, but also cross over between adjacent polyglactin filaments, as indicated in area 106. By crossing over, the polypropylene filaments are linked together along the y-axis, as well as extending along the length of the mesh along the x-axis. Thus, when the polyglactin filaments are absorbed, what remains is a mesh of polyproylene filaments that is continuous in both the x and y directions. In other words, the mesh that remains implanted maintains its full width construction and the scarring that invades the mesh is continuous throughout leaving a wide three-dimensional collagen fiber network. This structure ensures tissue ingrowth in a randomized manner along both the entire width and length of the mesh structure. As indicated above, such random ingrowth does not mimic the natural tissue structure of the tissue that is being reinforced or replaced, and may be unsuitable where narrow bands of tissue are to be replaced or reinforced, or where the tissue to be replaced requires more flexibility, especially in one particular direction.

The document GB1008193 A discloses a surgical mesh according to the preamble of claim 1.

Accordingly, there is a need for an improved implantable surgical mesh that reduces or alleviates the problems discussed above, and that promotes tissue ingrowth that more closely mirrors natural body tissue.

### SUMMARY OF THE INVENTION

An implantable surgical mesh is provided in accordance with claim 1.

In one embodiment, the plurality of absorbable and non-absorbable filaments are constructed in a woven configuration, and in another embodiment substantially all of the absorbable filaments are fill and substantially all of the non-absorbable filaments are wrap.

In an alternate embodiment, the plurality of absorbable and non-absorbable filaments are constructed in a knitted configuration. In further embodiments, the absorbable and non-absorbable filaments may alternate, the ratio of absorbable to non-absorbable filaments may be less or greater than 1:1.

The plurality of absorbable and non-absorbable filaments may alternatively be constructed in a combination knitted and woven configuration, or in a non-woven configuration.

In one embodiment, the non-absorbable filaments are selected from the group consisting of polypropylene, polyester, polyethylene, acrylic, polyamides, aramids, fluropolymer filaments, and flurocarbon filaments, and in yet another embodiment, the absorbable filaments are selected from the group consisting of polyglacting, polydioxanone, polycaprolactone, polylactic acid, and polylactide.

Also provided is an implantable surgical mesh having a plurality of absorbable filaments and a plurality of non-absorbable filaments, wherein substantially all of the non-absorbable filaments are arranged in rows which are aligned in a single direction with substantially no cross-linking therebetween, and wherein the plurality of absorbable filaments are arranged in rows which are aligned in a single direction and interwoven with the non-absorbable filaments to thereby form a bi-directional mesh structure prior to absorption of the absorbable filaments.

These and other features and advantages of the present invention will become apparent from the following more detailed description, when taken in conjunction with the accompanying drawings which illustrate, by way of example, the principles of the invention.

### BRIEF DESCRIPTION OF THE DRAWINGS

FIGURE 1 illustrates the configuration of a prior art mesh incorporating absorbable and non-absorbable fibers;
FIGURES 2a and 2b depict the pubocervical fascia within the pelvic cavity of a female;
FIGURE 3 illustrates one embodiment of a woven mesh according to the present invention;
FIGURE 4 illustrates an alternate embodiment of a woven mesh according to the present invention;
FIGURE 5 illustrates a third alternate embodiment of a woven mesh according to the present invention;
FIGURE 6 illustrates a fourth embodiment of a woven mesh according to the present invention;
FIGURES 7A and 7B illustrate alternate embodiments of a knitted mesh according to the present invention;
FIGURE 8A and 8B illustrate further embodiments of a knitted mesh according to the present invention;
FIGURE 9 illustrates yet another embodiment of a knitted mesh according to the present invention;
FIGURE 10 illustrates a combination woven and knitted mesh according to the present invention;
FIGURE 11 illustrates another embodiment of a combination woven and knitted mesh according to the present invention;
FIGURES 12a-c illustrate various embodiments wherein the absorbable and non-absorbable filaments are constructed in a non-woven configuration; and

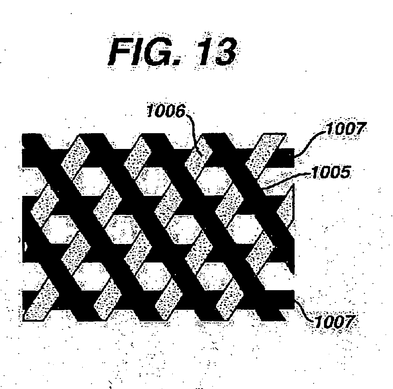

FIGURE 13 illustrates one embodiment of the present invention having a tri-axially woven configuration.

### DETAILED DESCRIPTION OF THE INVENTION

Before explaining the present invention in detail, it should be noted that the invention is not limited in its application or use to the details of construction and arrangement of parts illustrated in the accompanying drawings and description. The illustrative embodiments of the invention may be implemented or incorporated in other embodiments, variations and modifications, and may be practised or carried out in various ways. Further, although the present invention is primarily described in conjunction with pelvic floor repair procedures, it is to be understood that the invention and the principles described herein can be incorporated into any implantable surgical mesh used for any purpose. Some of those uses include but are not limited to, incontinence repair, ligament or smooth muscle repair in orthopedic procedures, cartilage repair for plastic surgery, or tissue replacement in orthopedic joints such as the meniscus of the knee and the labrum of the shoulder. Additional uses are for rebuilding smooth muscle within the abdominal or thoracic cavities because of loss due to trauma or disease.

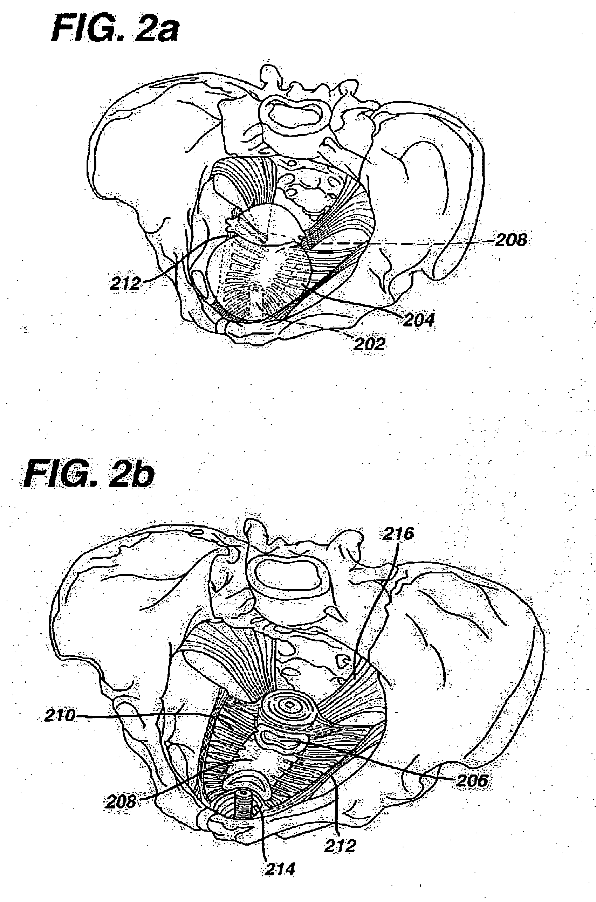

As was stated above, known implantable surgical meshes that incorporate absorbable and non-absorbable fibers leave behind (following absorption) a mesh structure that is continuous in both directions, thereby allowing randomized ingrowth substantially along the entire surface area of the mesh in a manner that does not approximate natural tissue growth. Referring now to Figs. 2a and 2b, the pubocervical fascia within the pelvic cavity of a female is shown in detail. These figures illustrate the pubocervical fascia relative to the pelvic bones and especially to the ischial spine and ischial tuberosity, as well as the pubic bone and obturator fossa rami, and also relative to the urethra 202, the bladder 204, the cervix 206, and the vagina 208. The horizontal portion of the pubocervical fascia 210 supports the bladder and vagina, and extends laterally from the tissue surrounding the vagina, outward to the fascial white line 212. The distal or vertical portion of the pubocervical fascia 214 supports the urethra and urethrovesical junction and provides a backstop against which the urethra is compressed during straining activity, such as coughing. As shown, the horizontal pubocervical fascia includes multiple striations that primarily extend laterally in the direction described above (between the fascial white line and the vaginal tissue), with very little cross-linking between these striations. Thus, in the natural state of the horizontal portion of the pubocervical fascia, the striations extend primarily in a single direction. The same is true for the vertical pubocervical fascia, and for the uterosacral ligaments 216.

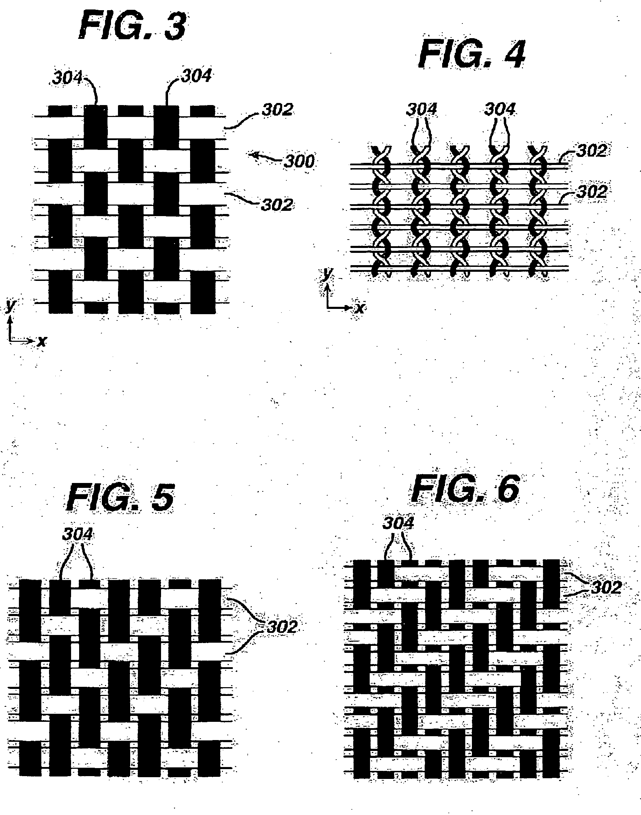

The present invention provides a mesh that will more closely resemble natural tissue structure, such as that of the pubocervical fascia. One embodiment of the present invention is illustrated in Fig. 3. The mesh 300 is a plain weave mesh including a plurality of absorbable filaments 302 positioned next to one another and extending along the width of the mesh in direction x, and a plurality of non-absorbable filaments 304 positioned next to one another and extending along the length of the mesh in direction y, and woven through the absorbable filaments. Thus, following absorption of the absorbable fibers, all that remains is a mesh structure of non-absorbable fibers that is continuous in a single direction, with substantially no cross-linking among the remaining fibers. The remaining mesh will have substantially flexibility in the x direction where there is no cross-linking, and less flexibility in the y direction where the non-absorbable filaments remain. Such a plain weave mesh can be manufactured by any well known technique, such as a shuttle loom, Jacquard loom or Gripper loom. In these looms the process of weaving remains similar, the interlacing of two systems of yams at right angles. This lacing can be simple as in a plain weave (Fig 3) where the lacing is over one and under one. Placing the absorbable yams in one direction, either fill (302) or wrap (304) (for each of Figs. 3-6 reference numeral 302 is used to denote absorbable fibers and 304 to denote non-absorbable fibers) will result in a final remaining product of the non-absorbent yams evenly spaced in one direction. Changing the plain weave to a more elaborate construction such as twill weave or satin weave, will provide different looks to the initial fabric, but as long as the absorbable material is laid-in in only one direction then the resultant product will still have yams attaching only on one end and no cross supports holding then together.

Another method of weaving is a leno weave. In this construction two warp yams are twisted and the fill yams are passed through the twist, Fig. 4. If the fill yarn302 is absorbable, and the fabric is made in an open construction there is some slack or spacing in the twisted warps 304. This can produce a resultant material, after the fills are re-absorbed, which has some elongation characteristics. The warps, however, are not connected and although they are individually embedded with scar tissue, there is no significant cross over of the scar tissue from yarn to yarn. It will be clear to those skilled in the art that additional variations of the basic weaves such as, sateen weaves, antique satin, warp faced twills (Fig 5) herringbone twills (Fig 6) and tri-axially woven fabrics as well as others can be used to create woven fabrics that will produce the same results when one of the directional yams absorbs.

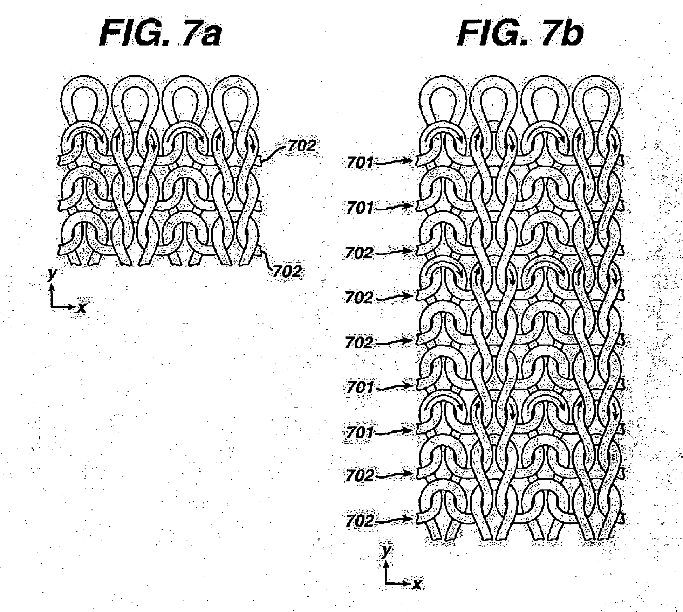

It is also possible to create fabrics using other manufacturing techniques, which will eventually produce a product, after some of the yams or filaments have absorbed, which is discontinuous and will provide support by connecting between two tissue areas, without significant connection between the yams. These fabrics are constructed by knitting, which is a process of making cloth with a single yarn or set of yams moving in only one direction. In weaving, two sets of yams cross over and under each other. In knitting, the single yarn is looped through itself to make the chain of stitches. One method to do this is described as weft knitting, an example of which is shown in Fig 7A. In this construction the yams are introduced from the side (the x direction) or horizontally opposite to the direction of growth of the fabric (the y direction). To create the discontinuous mesh, alternating yams (i.e., yams 702) would be absorbable yarns. The ratio of absorbable to non-absorbable yams can be adjusted to control the distance between the discontinuous portions of the mesh. Therefore, by laying-in multiple yams of absorbable and or non-absorbable material the width of the non-absorbable section can be controlled. This will provide different amounts of structural integrity of the remaining yams. As an example illustrated in Fig 7B, using two non absorbable yams 701 side by side, and three absorbable yams 702 side by side between them would produce a final fabric, after absorption, with larger space between the continuous yams and narrower width of the remaining material. Variations on this type construction will produce a remaining fabric, which promotes either more of less scar tissue depending on the amount of fabric and distance between sections. This can be adjusted for the type of tissue, which is being replaced. A lighter tissue, such as a fascia for supporting or connecting organs, can use a knitted mesh that has a wider section of absorbable and a narrower section of non-absorbable. A heavy tissue, such a ligaments for connecting bones across a joint, can have more non-absorbable yams and less or narrower absorbable portions.

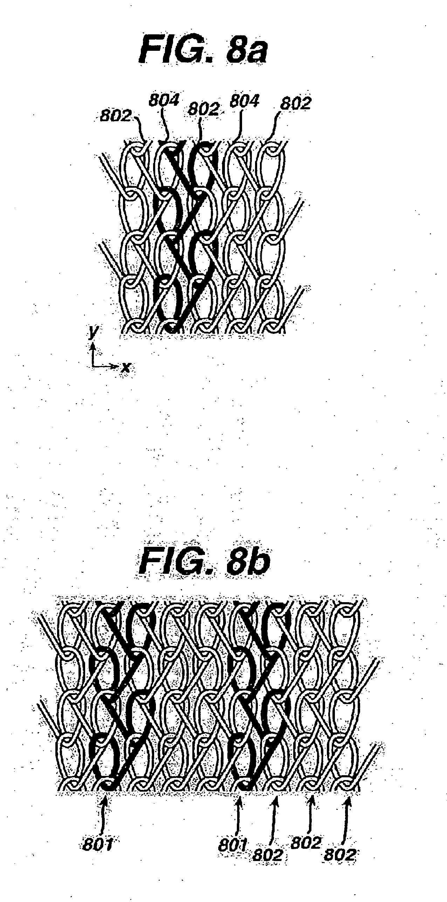

A second method for knitting a fabric or mesh is warp knitting. In this method the yams are introduced in the direction of the growth of the fabric (in the y direction) as is illustrated in Figs. 8A and 8B. In this type fabric the yams or filaments are looped vertically and also to a limited extent diagonally, with the diagonal movement connecting the rows of loops. As with the weft knit fabrics, alternate yams can be absorbable (i.e., 802) or non-absorbable (i.e., 804). Controlling the number and ratio of absorbable to non-absorbable yams will control the final material configuration and again the amount of in growth of scar tissue. In Fig 8A, alternating absorbable and non-absorbable yams produces a final construction with a narrow space between the remaining yarns which are filled in with tissue. By increasing the spacing between successive absorbable yams (as shown in Fig. 8B) the spacing between remaining yams can be selectively increased or decreased. In this manner, as with woven meshes, the warp knits can be adjusted to create various amounts of tissue creation and therefore can more closely emulate the tissue it is meant to replace.

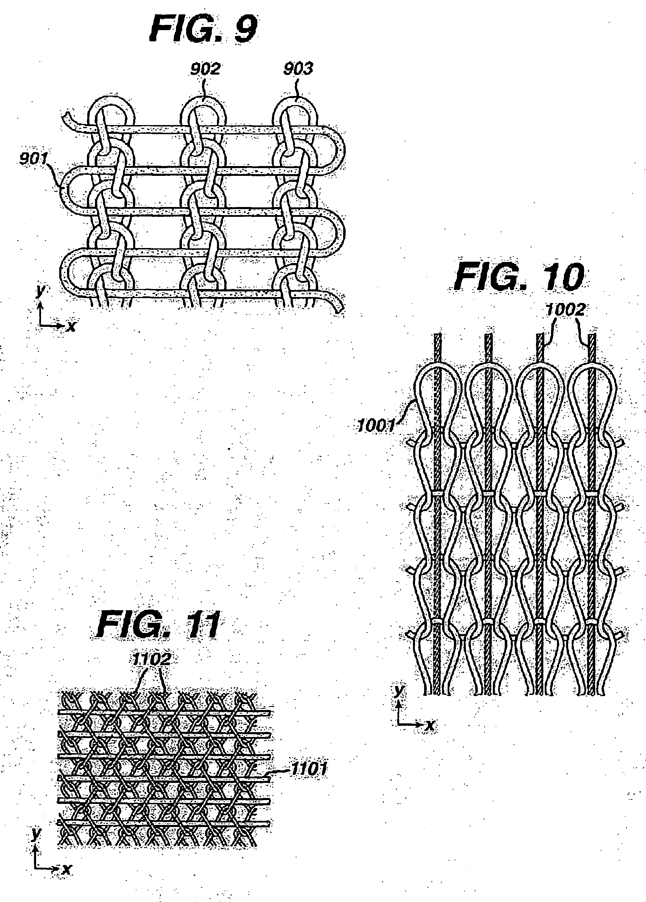

Different types of warp knits can be used to construct a fabric for this purpose, such as Tricots, Raschel and Cidega knits. In producing a warp knit with a Raschel knitting machine, multiple variations in construction can be achieved. Most will produce a fabric that will function essentially the same as described above. However, there is a technique in Raschel knitting that uses a "fall plate" that can produce a structure that will look more like a woven fabric, as shown in Fig.9. A single yarn 901 is carried across a number of warps, 902 and 903, in a horizontal or diagonal direction. This yarn connects and holds the warps together. When this yarn is made from an absorbable material and the warps are made from non-absorbable material, the final result after absorption will be only the warps aligned in the length direction with no connection between them. Again variations on the ratio of non-absorbable to absorbable material in the side by side warp yams can produce a resultant construction with the yams further apart or closer depending on how many warps are form either absorbable or non-absorbable material.

A third method of constructing a fabric consists of combining weaving and knitting. This method is called Co-We-Nit and is illustrated in Figs. 10 and 11. In this construction, knitting and weaving is combined to create fabrics with greater dimensional stability than conventional knits but with some of the properties of knitted goods. Starting with a weft knit shown in Fig. 10, the loop yams 1001 are fed from across the fabric, a straight strand or strands of yarns 1 002 are inserted in the opposite or warp direction (the y direction). These strands add stability to the knit in the vertical or warp direction, but do not affect the properties, such as elongation, in the weft direction. In the present invention, this fabric would have these laid-in warp yams as non-absorbable and the weft yams as absorbable. The resultant fabric would be easy to handle and position within the body, and provide a minimum of structure for the scar tissue to form around after the absorbable yams have gone. Spacing of these warp yams and the size or diameter would control the density of the remaining tissue. This same method can be used to produce a fabric from warp knitting as shown in Fig. 11, which will contain laid-in weft yams 1101 so that the stretch and elongation properties of the mesh in the warp direction (y direction) can be maintained in the initial construction, and then leave remaining a minimal structure after the absorbable warp yams 1102 have gone.

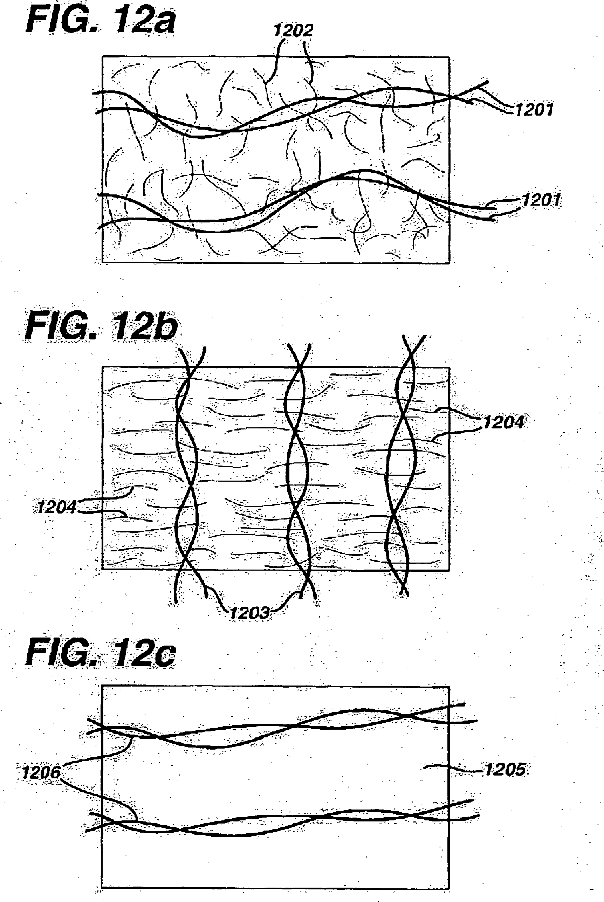

In alternate embodiments according to the present invention, the plurality of absorbable and non-absorbable filaments are constructed in a non-woven configuration. For example, Fig. 12a illustrates non-absorbable filaments spaced apart and positioned in a substantially uniform direction, with absorbable filaments 1202 being randomly oriented throughout. Fig. 12b illustrates non-absorbable filaments 1203 similarly positioned, but with the absorbable filaments 1204 positioned randomly, but substantially perpendicularly to the non-absorbable filaments. Finally, Fig. 12c illustrates a film or paper sheet 1205, such as a polydioxanone film or oxygen regenerated cellulose film, with non-absorbable filaments 1206 positioned spaced apart and in a substantially uniform direction. In the example of a sheet of paper, the fibers of the paper can be made from an absorbable material such as the oxygen regenerated cellulose, chopped into short filaments and then cast into a sheet. Other paper like constructions can include materials like poly vinyl alcohols, or collagen fibers derived from porcine or bovine sources.

Returning now to Figs. 2a and 2b, a mesh according to the present invention can be used to reinforced or replace the pubourethral ligament, or the horizontal portion of the pubocervical fascia, both of which have striations oriented primarily in a single direction as described above. With the mesh implanted so that the non-absorbable filaments are aligned with the natural striations, the remaining structure mimics the natural striations and allows flexibility in the opposite direction as does the natural ligament.

In a preferred embodiment, the absorbable filament is polygalactin and the non-absorbable filament is Polypropylene monofilament of 2.0 mils to 7.0 mils diameter, however, any suitable biocompatible absorbable and non-absorbable filaments could be used. It may be desirable to select a non-absorbable filament to control the desired structure integrity time, i.e. the time in which is takes for the filaments to absorb. The following table illustrates the approximate length of time it takes for various absorbable fibers to completely absorb:

| Fiber | Absorption Time | 0 lbs BSR |
|---|---|---|
| Polygalactin Vicryl | 90 days | 42 days |
| Polydioxanone PDS | 200 days | 90 days |
| Monocryl | 119 days | 28 days |
| Poly lactic acid Panacryl | 30 months | >200 days |
| Oxygen Regenerated Cellulose | 7 days | 2 days |
| Polycaprolactone | 40-90 days | 20-45 days |

The table above also illustrates the breaking strength (BSR) of these materials as compared to the absorption times. The BSR measures the time at which the material, in suture or filament form, will lose enough strength so that its tensile strength reaches essentially 0 lbs. Thus, the BSR more closely represents the loss of integrity of the structure.

In addition to selecting different materials, the diameter of the filaments can be selected to alter the physical properties of the mesh. For example, the absorbable filaments may be of smaller, or larger diameters than the non-absorbable filaments. Increasing the diameter of the filament can increase the absorption time as well.

Fig. 4 shows another embodiment of the present invention. A mesh 400 includes a plurality of helically coiled non-absorbable filaments 402 extending the length of the mesh in the x direction, but which are separate and not interwoven with one another. A plurality of non-absorbable filaments are positioned between successive absorbable filaments, but are also woven through the non-absorbable filaments on either side. In this manner, the absorbable filaments are part of the structure of the mesh and provide structural integrity for the mesh in the y direction. When the absorbable filaments are completely absorbed, however, what remains is only the non-absorbable filaments extending in the x direction with no binding together or cross-linking of adjacent filaments.

Although specific embodiments of the invention have been described herein, it is to be understood that any weave or knit patterns, or non-woven patterns, in which the absorbable filaments dissolve or are absorbed to leave behind a substantially unidirectional mesh structure is within the scope of the invention. Further, although the described embodiments show no interweaving among successive non-absorbable filaments, some cross-weaving can take place and still provide a mesh with substantially uni-directional filaments. For example, in Fig 10 one or two rows of the weft yams 1001 can be non-absorbable and then 5 to 10 rows can be absorbable. The resultant fabric will have a loose connection between the warp yams 1002. In these types of fabrics, yams can also be laid in a diagonal direction, thereby creating a structure that has permanent support in a third alternate direction. In Tri-axially woven fabrics, the absorbable warp yams 1005, 1006 are set in at two diagonal directions with the non-absorbable fill yams 1007 extending substantially parallel to one another in a single direction, as shown in Fig 12. Changing the absorbable and non-absorbable yams from the fill to the warp, either both or one, provides yet another construction, which when the absorbable warp yams resorb, yields a discontinuous structure, consisting of only the remaining non-absorbable fill yarns.

In yet another embodiment of the concept, the construction of the fabric can made from a non-woven process. In the non-woven process, filaments are mechanically deposited to form a mat. The mat is then treated to provide integrity. The treatment can include manipulation of the filaments to entangle them or melt them together, or bind them with an adhesive or curing resin. In this example, alternate strips of the mat can be composed of non-absorbable and absorbable material such that as the absorbable material absorbs and the mat structure becomes discrete strips of material. These remaining strips will be in grown with tissue and provide a unidirectional support for the tissue. As with the weaves or knits described above, yarns of non-absorbable material may be laid in in the non-woven fabric. If the deposited filaments are absorbable and are bound together either through mechanical, thermal or chemical methods, then as they dissolve, the non-absorbable yams will remain and provide the structure for tissue in growth. As described above, these yams can be interlaced as well as linear. Further, they can be in a sinusoidal pattern or other side to side type pattern, and can be in the machine (warp) direction, cross (weft or fill) direction or diagonal, so long as they provide permanent connection of the remaining structure to the surrounding tissue, and provide a support for the tissue as well as a scaffold for the tissue to grow on and in.

An additional method to create a structure which will have a continuous construction initially, and then a discontinuous structure after some of the material has dissolved is to build a lamination of different materials. In this example a sheet of absorbable material such as oxygenated regenerated cellulose (ORC) can be laminated to filaments of a non-absorbable material such as polypropylene. The sheet can be produced with a wet lay process such as in the manufacture of papers, or a dry lay process such as in the manufacture of felts or non-wovens, or as a film. Once the structure is placed in the body the ORC material will dissolve within a few days leaving the polypropylene in place. The polypropylene elicits a foreign body response and inflammation. The inflammation leads to fibrotic activity and the cascade of scarring occurs. Scar tissue forms around the polypropylene filaments covering them through their length but not producing a significant amount of cross over between them. This then produces an essentially discontinuous configuration of scar tissue.

Depending on the distance between the filaments, usually greater than 1000 microns, scar formation will not bridge across the gap. However some light tissue formation may occur. This may even be encouraged by crossing a very few filaments, either in the opposite direction, or by allowing some filaments to curve or wind enough to reach others. In this way building a support mechanism for injured or diseased tissue can be precisely controlled to match the original tissue in thickness and flexibility properties.

Although several embodiments of a mesh for pelvic floor prolapse repair have been described, those skilled in the art will recognize that various other mesh configurations can also be used in conjunction with the procedures and techniques described herein. It will be further apparent from the foregoing that other modifications of the inventions described herein can be made without departing from the scope of the invention. Accordingly, it is not intended that the invention be limited, except as by the appended claims.

## Claims

1. An implantable surgical mesh (300) comprising:
a plurality of absorbable filaments (302); and
a plurality of non-absorbable filaments (304);
wherein the plurality of absorbable filaments are interwoven with the non-absorbable filaments to thereby form a mesh structure prior to absorption of the absorbable filaments, **characterised in that** substantially all of the non-absorbable filaments are substantially aligned in a single direction with substantially no cross-linking therebetween and **in that** the mesh structure is bi-directional.

2. The implantable surgical mesh according to claim 1, wherein the plurality of absorbable and non-absorbable filaments are constructed in a woven configuration.

3. The implantable surgical mesh according to claim 2, wherein substantially all of the absorbable filaments are fill and substantially all of the non-absorbable filaments are wrap.

4. The implantable surgical mesh according to claim 1, wherein the plurality of absorbable and non-absorbable filaments are constructed in a knitted configuration.

5. The implantable surgical mesh according to claim 4, wherein absorbable and non-absorbable filaments alternate.

6. The implantable surgical mesh according to claim 4 or claim 5, wherein the ratio of absorbable to non-absorbable filaments is less than 1:1.

7. The implantable surgical mesh according to claim 4 or claim 5, wherein the ratio of absorbable to non-absorbable filaments is greater than 1:1.

8. The implantable surgical mesh according to claim 1, wherein the plurality of absorbable and non-absorbable filaments are constructed in a combination knitted and woven configuration.

9. The implantable surgical mesh according to claim 1, wherein the plurality of absorbable and non-absorbable filaments are constructed in a non-woven configuration.

10. The implantable surgical mesh according to any one of claims 1 to 9, wherein the non-absorbable filaments are polypropylene, polyester, polyethylene, acrylic, polyamide, aramid, fluoropolymer or fluorocarbon filament.

11. The implantable surgical mesh according to any one of claims 1 to 10, wherein the absorbable filaments are polyglactin, polydioxanone, polycaprolactone, polylactic acid or polylactide filaments.

12. The implantable surgical mesh according to claim 1,
wherein the non-absorbable filaments (304) are arranged in rows ; and
wherein the plurality of absorbable filaments (302) are arranged in rows which are aligned in a single direction.

## Patentansprüche

1. Implantierbares chirurgisches Netz (300), das aufweist:
eine Vielzahl absorbierbarer Filamente (302); und
eine Vielzahl nicht absorbierbarer Filamente (304);
wobei die Vielzahl der absorbierbaren Filamente mit den nicht absorbierbaren Filamenten verflochten ist, um somit vor der Absorption der absorbierbaren Filamente eine Netzstruktur zu bilden, **dadurch gekennzeichnet, daß** im wesentlichen alle nicht absorbierbaren Filamente im wesentlichen in einer einzigen Richtung im wesentlichen vernetzungsfrei ausgerichtet sind und daß die Netzstruktur bidirektional ist.

2. Implantierbares chirurgisches Netz nach Anspruch 1, bei dem die Vielzahl der absorbierbaren und der nicht absorbierbaren Filamente in einer gewebten Ausgestaltung aufgebaut sind.

3. Implantierbares chirurgisches Netz nach Anspruch 2, bei dem im wesentlichen alle absorbierbaren Filamente Füller sind und wesentlich alle nicht absorbierbaren Filamente Windungen sind.

4. Implantierbares chirurgisches Netz nach Anspruch 1, bei dem die Vielzahl der absorbierbaren und nicht absorbierbaren Filamente in einer gestrickten Ausgestaltung aufgebaut sind.

5. Implantierbares chirurgisches Netz nach Anspruch 4, bei dem sich absorbierbare und nicht absorbierbare Filamente abwechseln.

6. Implantierbares chirurgisches Netz nach Anspruch 4 oder Anspruch 5, bei dem das Verhältnis von absorbierbaren zu nicht absorbierbaren Filamenten kleiner als 1:1 ist.

7. Implantierbares chirurgisches Netz nach Anspruch 4 oder Anspruch 5, bei dem das Verhältnis von absorbierbaren zu nicht absorbierbaren Filamenten größer als 1:1 ist.

8. Implantierbares chirurgisches Netz nach Anspruch 1, bei dem die Vielzahl der absorbierbaren und der nicht absorbierbaren Filamente in einer Kombination aus gestrickter und gewebter Ausgestaltung aufgebaut ist.

9. Implantierbares chirurgisches Netz nach Anspruch 1, bei dem die Vielzahl der absorbierbaren und der nicht absorbierbaren Filamente in einer vliesartigen Ausgestaltung aufgebaut ist.

10. Implantierbares chirurgisches Netz nach einem der Ansprüche 1 bis 9, bei dem die nicht absorbierbaren Filamente Polypropylen-, Polyester-, Polyethylen-, Acryl-, Polyamid-, Aramid-, Fluorpolymer- oder Fluorkohlenstoff-Filament sind.

11. Implantierbares chirurgisches Netz nach einem der Ansprüche 1 bis 10, bei dem die absorbierbaren Filamente Polyglactin, Polydiaoxanon, Polycaprolacton, Polymilchsäure oder Polylactidfilamente sind.

12. Implantierbares chirurgisches Netz nach Anspruch 1,
bei dem die nicht absorbierbaren Filamente (304) in Zeilen angeordnet sind; und
bei dem die Vielzahl der absorbierbaren Filamente (302) in Zeilen angeordnet sind, die in eine einzige Richtung ausgerichtet sind.

## Revendications

1. Treillis chirurgical implantable (300) comprenant :
◆ une pluralité de filaments absorbables (302) ; et
◆ une pluralité de filaments non absorbables (304) ;
dans lequel la pluralité de filaments absorbables est entrelacée avec les filaments non absorbables pour former ainsi une structure en treillis avant l'absorption des filaments absorbables,
**caracterisé en ce que** pratiquement tous les filaments non absorbables sont pratiquement alignés dans une seule direction, pratiquement sans aucune liaison entre eux, et **en ce que** la structure en treillis est bidirectionnelle.

2. Treillis chirurgical implantable selon la revendication 1, dans lequel la pluralité de filaments absorbables et non absorbables est agencée selon une configuration tissée.

3. Treillis chirurgical implantable selon la revendication 2, dans lequel pratiquement tous les filaments absorbables sont de la matière de remplissage et pratiquement tous les filaments non absorbables sont une enveloppe.

4. Treillis chirurgical implantable selon la revendication 1, dans lequel la pluralité de filaments absorbables et non absorbables est agencée selon une configuration tricotée.

5. Treillis chirurgical implantable selon la revendication 4, dans lequel les filaments absorbables et non absorbables sont alternés.

6. Treillis chirurgical implantable selon la revendication 4 ou 5, dans lequel le rapport entre les filaments absorbables et non absorbables est inférieur à 1:1.

7. Treillis chirurgicale implantable selon la revendication 4 ou 5, dans lequel le rapport entre les filaments absorbables et non absorbables est supérieur à 1:1.

8. Treillis chirurgical implantable selon la revendication 1, dans lequel la pluralité de filaments absorbables et non absorbables est agencée selon une combinaison tricotée et tissée.

9. Treillis chirurgical implantable selon la revendication 1, dans lequel la pluralité de filaments absorbables et non absorbables est agencée selon une configuration non tissée.

10. Treillis chirurgical implantable selon l'une quelconque des revendications 1 à 9, dans lequel les filaments non absorbables sont en polypropylène, en polyester, en polyéthylène, en acrylique, en polyamide, en aramide, en fluoropolymère ou en fluorocarbone.

11. Treillis chirurgical implantable selon l'une quelconque des revendications 1 à 10, dans lequel les filaments absorbables sont des filaments en acide polyglactine, en polydioxanone, en polycaprolactone, en acide polylactique ou en polylactide.

12. Treillis chirurgical implantable selon la revendication 1, dans lequel les filaments non absorbables (304) sont disposés en rangées ; et dans lequel la pluralité de filaments absorbables (302) est disposée en rangées qui sont alignées dans une seule direction.
